Europäisches Patentamt

European Patent Office

Office européen des brevets

(19)

(11) **EP 0 561 789 B1**

(12) **EUROPEAN PATENT SPECIFICATION**

(45) Date of publication and mention
of the grant of the patent:
**17.04.1996 Bulletin 1996/16**

(21) Application number: **91917609.9**

(22) Date of filing: **29.08.1991**

(51) Int Cl.$^6$: **G01N 21/35**

(86) International application number:
**PCT/US91/06193**

(87) International publication number:
**WO 92/10738 (25.06.1992 Gazette 1992/14)**

(54) **DETERMINATION OF AROMATICS IN HYDROCARBONS BY NEAR INFRARED SPECTROSCOPY**

BESTIMMUNG DER AROMATEN IN DEN KOHLENWASSERSTOFFEN DURCH SPEKTROMETRIE IM NAHEN INFRAROT

DETERMINATION DES AROMATIQUES PRESENTS DANS DES HYDROCARBURES A L'AIDE DE LA SPECTROSCOPIE A INFRAROUGE PROCHE

(84) Designated Contracting States:
**DE FR GB IT**

(30) Priority: **11.12.1990 US 626132**

(43) Date of publication of application:
**29.09.1993 Bulletin 1993/39**

(73) Proprietor: **ASHLAND OIL, INC.**
**Ashland, KY 41114 (US)**

(72) Inventors:
• **MAGGARD, Steven, M.**
**Barboursville, WV 25504 (US)**
• **WELCH, William, T.**
**Ashland, KY 41101 (US)**

(74) Representative: **Lambert, Hugh Richmond et al**
**D. YOUNG & CO.,**
**21 New Fetter Lane**
**London EC4A 1DA (GB)**

(56) References cited:
EP-A- 0 285 251          EP-A- 0 304 232
EP-A- 0 305 090          GB-A- 2 217 838
US-A- 4 800 279          US-A- 4 963 745

## Description

The present invention relates to the determination of aromatic constituents in hydrocarbons by near infrared (NIR) spectroscopy.

Hydrocarbon analysis by NIR spectroscopy is known and is used for a variety of different purposes. For example US-A-4,963,745 discloses the use of NIR spectroscopy to measure the octane rating of gasoline. See also "Prediction of Gasoline Octane Numbers from Near Infrared Spectral Features in the Range 660-1215 nm" by J.J. Kelly et al., Analytical Chemistry, vol. 61, no. 4, February 15, 1989, pp 313-320, and "Nondestructive Analytical Procedure for Simultaneous Estimation of the Major Classes of Hydrocarbon Constituents of Finished Gasolines", Analytical Chemistry, vol. 62, no. 14, July 15, 1990, pp 1444-1451.

Also, in GB-A-2,217,838 NIR spectroscopy is used to determine a variety of petrophysical and petrochemical properties of hydrocarbon mixtures, and more particularly teaches the use of the NIR absorbances measured at three different wavelengths as a secondary calibration technique to correlate the NIR absorption of a test sample to a primary standard and using multivariate analysis of the absorbance data, as taught, for example, in "Near Infrared Reflectance Analysis by Gauss-Jordan Linear Algebra", D.E. Honigs, J.M. Freelin, G.M. Hieftje, T.B. Hirschfeld, Applied Spectroscopy, vol. 37, no. 6, 1983, pp 491-497.

Other NIR analysis techniques for other purposes are taught, for example, in J. Prakt. Chem., 317(1), 1-16 where NIR is used to perform structural group analysis of mixtures of saturated and aromatic hydrocarbons, and in Leimer and Schmidt in Chem. Tech. (Leipzig), 25(2), 99-100, where NIR is used in the quantitative analysis of benzene-toluene-paraffin mixtures.

NIR spectroscopy has also been used in the determination of hydrocarbon functional groups such as $CH_3$ and $CH_2$, see Tosi and Tinto, Spectrochim ACTA, Part A, 28(3), 585-97.

Ultraviolet and near-infrared analysis of mixtures of aromatics is also taught by schmidt in Erdoelkohle, Erdgas, Petrochem., 21(6), 334-40, who sought to determine concentrations of specific compounds.

Each of the above references teach as a correlation between the NIR absorbance and some physical or chemical property of the mixture under investigation but none of them teach as an absolute aromatic determination for a very complex mixture such as diesel fuel as is involved in the present invention.

Also none of the references teach the determination of aromatics in a hydrocarbon mixture where GC separation is not possible due to coelution, GC separation being one of the more commonly used techniques in the analysis of hydrocarbons and where the percents of each of the individual compounds of a hydrocarbon mixture and as detected by gas chromatography are grouped under their respective generic classifications in the Paraffins, Isoparaffins, Aromatics, Naphthalenes, Olefins (PIANO) classification system, and the relative percentage of each of the components paraffins through olefins is determined in weight percent, volume percent, or mole percent as required. An example of this procedure is that taught by Analytical Automation Specialists, Inc., "The Detailed Analysis of Petroleum Naphthas, Reformates, Gasoline and Condensates by High-Resolution Gas Chromatography", Operators Manual, P.O. Box 80653, Baton Rouge, Louisiana 70898. Also available is AAS (Analytical Automated Systems) PIANO Software Package, Sievers Research PIANO Software Package.

Increasing governmental regulation and environmental laws are impacting the permissible percentage of aromatics tolerated in diesel fuels, turbine fuels, kerosene, heating oils, and other oils.

Therefore the need for accurate, absolute determinations of the present invention, particularly for on-line control of aromatics in fuels is increasing rapidly.

According to the present invention NIR spectroscopy is used to determine the absolute concentration of aromatics in hydrocarbon mixtures especially diesel fuel, kerosene, fuel oils at all grades such as light cycle oil, light vacuum oil, heating oils and vacuum oils as known in the petroleum industry. More particularly by measuring the absorption at a selected frequency or frequencies in the range 1650-1700 or 2120-2256 nm, and correcting for the absorption due to non-aromatics in the sample at that frequency, the absolute value of (or percent) aromatics in the sample can be determined.

The establishment of the standard by which the NIR absorbance at those selected wavelengths is compared in order to derive the aromatic content of the mixture from those measurements is an important feature of the present invention and is accomplished by preliminarily separating the aromatics from the non-aromatics in the sample or a sample of similar composition by use of well-known preparative high performance liquid chromatography, e.g. as described in Petroleum Derived Hydrocarbons, John D. Bacha, John W. Newman, and J.L. White, ACS Symposium series 303, Chapter 6.

The NIR absorbance of each of the two portions is measured at the selected frequency or frequencies (e.g. 1672 nm), and the absorbances of those two fractions are used to determine the equation to be used in deriving the aromatic content of the original mixture, or of similar mixtures to be analysed subsequently. Thereafter the solution of the equation:

$$Y = MX + b + e$$

can be used to determine the percent aromatics of many successive samples so long as the molecular constituents remain approximately the same. Thus, using the internal standardisation technique of this invention, only one standardisation is needed for a long period and many analyses.

In the above equation:

M =   the slope of the line (by the standard)
X =   the absorbance of the aromatics
b =   a constant which is determined by the fitting of the absorbance against the absolute weight values obtained above
Y =   percent aromatics
e =   any error in the determination

Therefore the characteristic elements of the invention may be stated as:

(a) separating the aromatics from the non-aromatics (saturates and olefins) in a representative sample to be used as a calibrating standard, preferably by preparatory high performance liquid chromatograph ("prep.HPLC"), said sample being taken from the mixture to be analysed, or one of similar composition;
(b) measuring NIR absorbance of the resulting non-aromatics and aromatic portions derived from the above sample;
(c) using the absorbances measured in (b) to derive the calibration equation and its constants by Beer-Lambert's Law or other well-known spectral data resolution techniques;
(d) measuring the NIR absorbance of a mixture to be analysed; and
(e) using the calibration equation, as determined in (a) and the NIR absorbance as determined in (d) to determine the aromatic content of the mixture (or conversely the non-aromatic content).

The invention as described above, can be utilised for most fuels, preferably for diesel fuel, especially in the diesel No. 2 fuel oil boiling range and preferably by measuring absorbance at a selected frequency or frequencies in the range 1650-1700 nm or 2120-2256 nm. The invention can be utilised as a batch process, in a flow-through cell, by the use of fibreoptic probes either bundled or single fibre, and the process control can be either feed-back or feed-forward based on the samples absorbance in the near-infrared, or optionally the first derivative of the samples absorbance or some other mathematical function of absorbance, being employed e.g. to operate a control valve.

In the drawings:

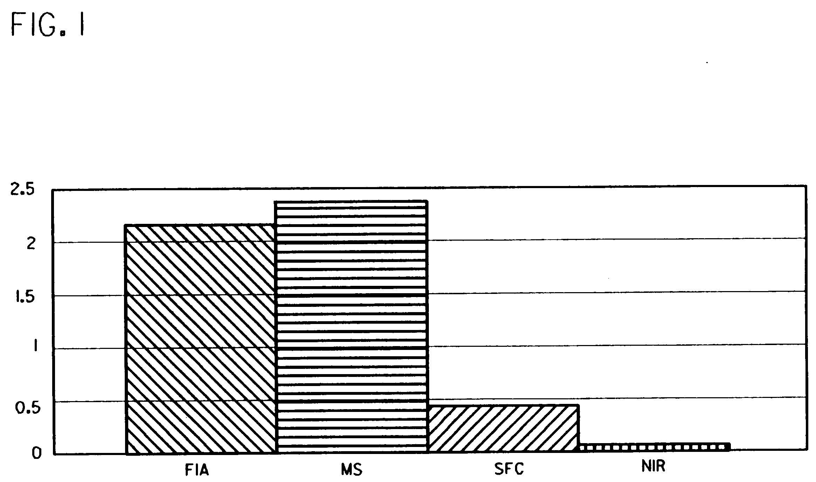

Figure 1 is a comparison of the repeatability error (% by absolute error) of four analytical methods for determining aromatics in diesel fuels (no. 2 fuel oil). The methods compared are fluorescent indicator absorption (FIA) by ASTM D-1319; mass spectroscopy (MS); super fluid chromatography (SFC) in which $CO_2$ above its critical pressure and temperature is used as the eluent; and near infrared according to the techniques of the present invention (NIR) for sets of 6 identical samples. This figures shows the excellent repeatability of the techniques of the present invention. (All determinations are in wt. % except FIA is in vol.%.).

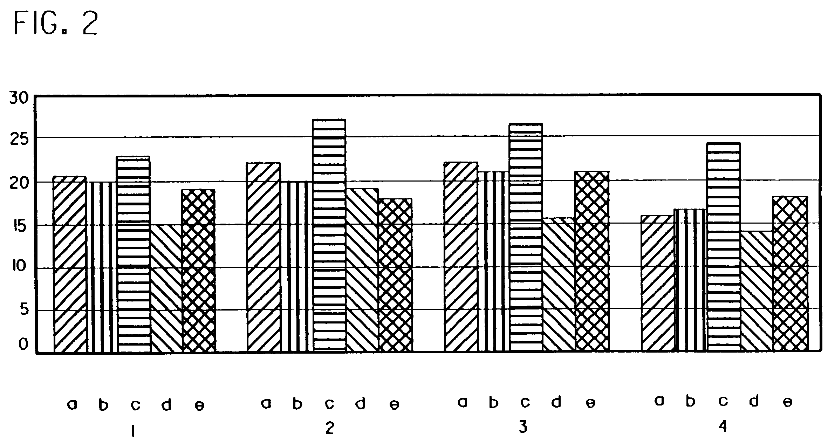

Figure 2 is a comparison of the percent aromatics determined in identical sets of 4 samples by heated FIA (a), heated FIA plus water deactivation of the chromatographic column (b), SFC (C); HPLC/DCD (high performance liquid chromatograph using a dielectric constant-measuring detector) (d); and the NIR of the present invention (e). Note that results are expressed in volume percent for the two FIA methods and the HPLC/DCD method, whereas SFC and NIR results are expressed in weight percent. Note that NIR (except in sample no. 2) is closest to the average of all methods combined.

Figure 3 is a schematic diagram of a control system utilizing the NIR techniques of the present invention to control a refinery hydrogenation unit in which sufficient hydrogen is added not only to combine with the sulphur, but also sufficient to cause scission and destruction of aromatics in diesel fuel.

Figure 4 is a near infrared absorbance spectrum of the aromatic fraction of a diesel fuel which was obtained by HPLC.

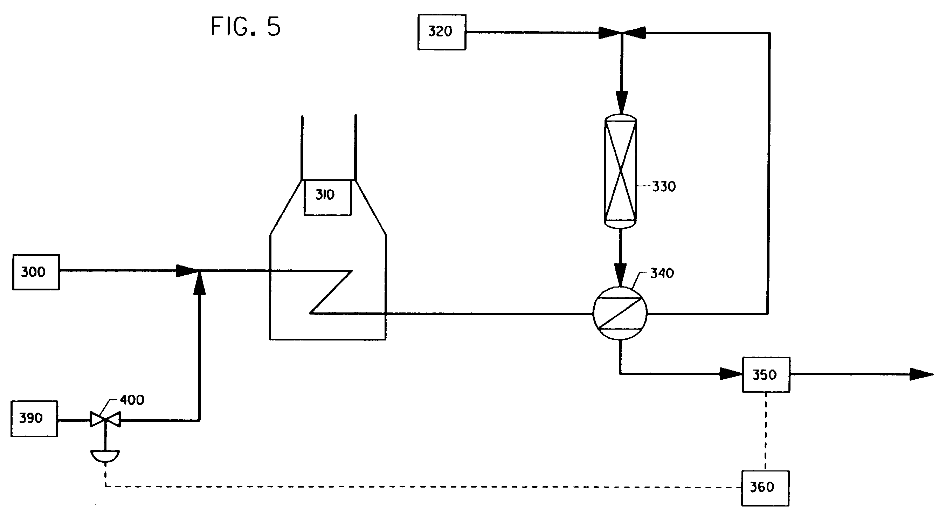

Figure 5 shows a schematic control system as detailed in Example 4.

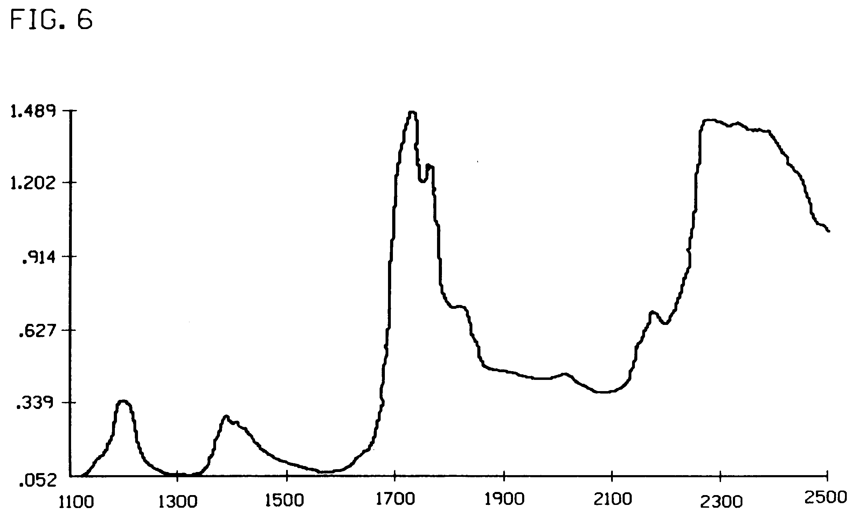

Figure 6 is a near infrared absorbance spectrum of a typical diesel fuel.

Figure 7 is a plot showing the correlation observed at each wavelength from 1100 to 2500 nm for the aromatic content of diesel fuels using the techniques of the current invention. Note the superior correlation discovered at 1650 to 1700 nm and at 2120 to 2256 nm, most preferably 1654 to 1696 nm, or 2124 to 2252 nm, or both.

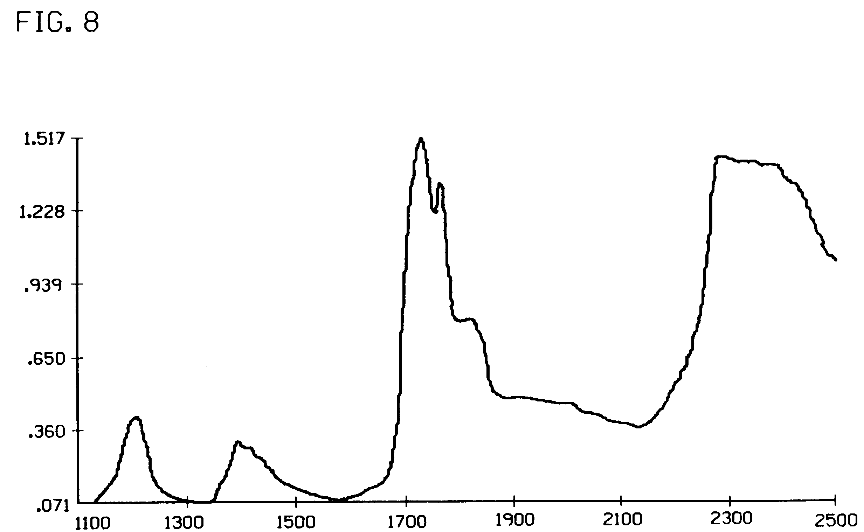

Figure 8 is a near infrared absorbance spectrum of the non-aromatic fraction of diesel fuel which was obtained by HPLC. The method of this invention, and several of its applications, are described in the following Examples.

## EXAMPLE 1

A diesel fuel sample (6 grams) is separated into its aromatic and non-aromatic fractions by passing (@ 500 ml/minute) the fuel down a silica diamine column connected in series to a silica gel column using hexane as the solvent on a Waters Div. Millipore, Milford, Massachusetts, Model 500A high performance liquid chromatograph. The saturate fraction is collected off the end of the column and the hexane mobile phase is removed by rotary evaporation. The aromatics are then back flushed from the column by reversing the flow and substituting methylene chloride as the solvent. The solvent is again removed by rotary evaporation.

The near infrared absorbance spectra of the non-aromatic and aromatic fractions (or known proportions thereof) are measured on an NIR Systems, Inc. Model 6500 spectrophotometer at 1672 nm and assigned concentrations of 0% and 100% aromatics by weight, respectively.

An equation of the form Y = mX + b is calculated from Beer-Lambert Law, where Y is the aromatic concentration, X is the absorbance, m is the slope of the regression line, and b is its Y-intercept.

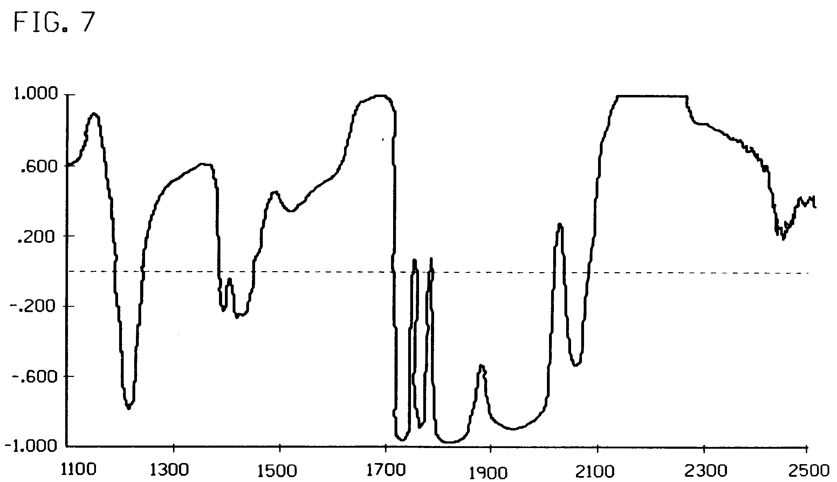

The absorbances of a series of five unknown diesel fuel samples are measured and, using the above equation and the sample's absorbance at 1672 nm, aromatic concentration of each sample is calculated and the correlations are plotted together as Figure 7. Note the near perfect correlation in the preferred bands of the invention.

## EXAMPLE 2

When the techniques of Example 1 are employed using fibreoptic probes to measure flowing streams and side streams in a refinery diesel fuel stream, accuracy approximately as good as obtained in the batch-process of Example 1 is achieved. Fibre optical probes preferably use the NIR range of 1650 to 1700 nm because of the cost and difficulty in obtaining non-absorbing fibre probes which transmit in the range of 2124 to 2252 nm.

## EXAMPLE 3

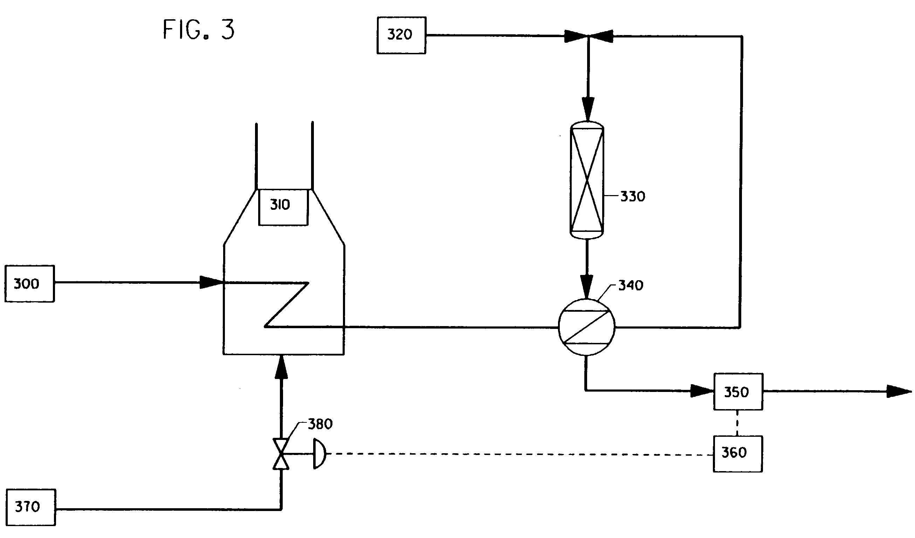

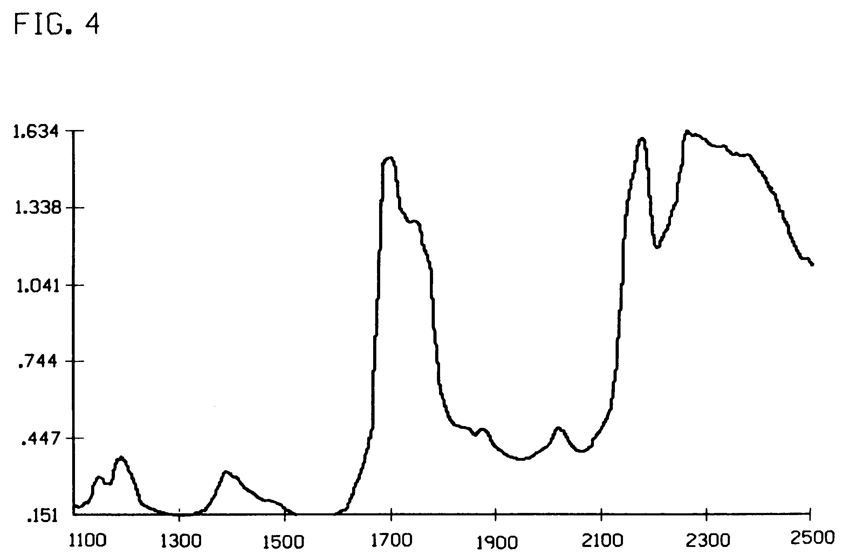

Figure 3 schematically shows an important process control application of this invention. New diesel fuel regulations require that highway fuel produced after October 1993 met a minimum cetane number specification. Cetane number and aromatics are closely related, as shown for Example in Figure 4. "Reduction of Aromatics in Diesel Fuel", A.J. Suchanek, in National Petroleum Refiners Association, AM-90-21, 1990. By use of this or similar relations, and an NIR instrument calibrated as according to this invention, very close control of hydrotreater operation can be attained at minimum severity and hydrogen consumption. Raw feed, stream 300, flows through fired heater 310, fwel/effluent exchanger 340, is admixed with hydrogen stream 320, through an adiabatic hydrotreating reactor 330, to tankage. Aromatic content of the stream is measured by NIR probe 350, and this information provided to the process control computer 360. Conversion of aromatics content to estimated cetane number is made by the computer, and the flow of fuel stream 370 to the fired heater is adjusted as necessary by control valve 380. For instance, aromatics levels are higher and cetane number is lower in the fired heater and at lower reactor temperature. This lower temperature reduces the aromatics saturation reaction rate, increasing the aromatic content (decreasing the cetane number) of the diesel product. This results in a savings in hydrogen consumption, fuel, and other utilities. In the case of measured cetane number less than 40, a similar chain of events results in higher hydrotreating severity and reduced in the effluent product aromatics. This on-line control allows rapid, very lose control of product quality and eliminates present problems with production of off-spec finished products in quantities because corrections are implemented much sooner. This also results in a close-specification diesel fuel which is in much demand in today's marketplace.

## EXAMPLE 5

Manufacture of Low Odor Base Solvent (LOBS) is accomplished by the control schematic of Figure 3. An NIR probe, properly calibrated for 0 to 5% aromatics, is used to directly control product quality (low aromatic content) to meet the specification level, by hydrotreatment. Blending also can be controlled by NIR, preferably using feed-forward of the aromatics content of the feed as measured by NIR.

The specific compositions, methods and embodiments described herein are intended to be merely illustrative of the invention disclosed by this specification. Variation on these compositions, methods, or embodiments will be readily apparent to a person of skill in the art, based upon these teachings, and are therefore to be included as within the scope of the invention hereinbefore described, and hereinafter claimed.

## Claims

1. A process for the measurement of the aromatics content of a liquid hydrocarbon mixture containing both aromatic and non-aromatic components, which comprises the steps of a) measuring the near infrared absorbance of the mixture at a selected wavelength or wavelengths, and b) utilising the absorbance value or values obtained in a) to generate an output signal indicative of the aromatic content of the mixture, characterised by the steps of

   i) obtaining a preliminary sample having the same composition as the mixture to be analyzed, or a composition similar thereto;
   ii) separating that preliminary sample into an aromatic fraction and a non-aromatic fraction;
   iii) measuring the infrared absorbance of those fractions, each at the same selected wavelength or wavelengths in the range 1650 to 1700 nm or 2120 to 2256 nm;
   iv) using the absorbance values measured in iii), to derive the calibration equation to be used in the subsequent analysis;
   v) measuring the infrared absorbance of the mixture to be analyzed at the same selected wavelength or wavelengths; and
   vi) using the calibration equation derived in step iv) to determine the aromatic content of that mixture.

2. A method according to claim 1, wherein, in step i) of the pre-calibration process, the said sample is of the same composition as the mixture to be analyzed and is obtained by pre-sampling of that mixture.

3. A process according to claim 1, wherein the pre-calibration process is effected using a sample taken from a different hydrocarbon mixture from the mixture that is to be analyzed but nevertheless being of a similar composition.

4. A process according to any one of claims 1 to 3, wherein, in step iv), the absorbance values obtained in step iii) are used to derive the calibration equation by the application of Beer-Lambert's law.

5. A process according to any one of claims 1 to 4, wherein, in step a) of the measurement process and step iii) of the pre-calibration process, the absorbances are measured at a single wavelength within said ranges 1650 to 1700 nm and 2120 to 2256 nm.

6. A process according to claim 5, wherein that single wavelength is 1672 nm.

7. A process according to any one of claims 1 to 6, wherein, in step ii) of the calibration process, the said sample is separated into said aromatic and non-aromatic fractions by high performance liquid chromatography.

8. A process according to any one of claims 1 to 7, wherein the absorbance measurement of step a) is carried out on a substantially continuous basis whilst the hydrocarbon mixture to be analyzed, or a portion thereof, is flowing through a through flow cell designed to permit infrared absorbances to be measured on a liquid flowing through that cell, the signal generated in step b) thus being a substantially instantaneous aromatic content of the liquid hydrocarbon mixture as it flows through that cell.

9. A method of treating a liquid hydrocarbon feedstock which involves treating the feedstock in a manner to affect the aromatic content of that feedstock, wherein the aromatic content of the feedstock, before or after the said treatment, is monitored by means of a process claimed in claim 8, and wherein one or more process parameters affecting the aromatic content of the feedstock, after treatment, is adjusted in response to the output signal indicative of the instantaneous aromatic content of the feedstock at the point of measurement.

10. A method according to claim 9 which involves hydrotreating a liquid hydrocarbon feedstock in order to reduce its aromatic content, and wherein the said monitoring is effected on the effluent from the hydrotreater, the output signal indicative of the aromatic content of that effluent being used to adjust one or more of the parameters affecting the hydrotreatment and thereby to control the hydrotreatment process.

11. A method according to claim 10 as applied to the control of the cetane number of a diesel fuel, being produced by the hydrotreatment of a diesel fuel feedstock.

**Patentansprüche**

1. Verfahren zur Messung des Gehalts an aromatischen Verbindungen eines flüssigen Kohlenwasserstoff-Gemisches, das sowohl aromatische als auch nichtaromatische Komponenten enthält, umfassend die Stufen a) Messung der Absorption im nahen Infrarotbereich des Gemisches bei einer ausgewählten Wellenlänge oder ausgewählten Wellenlängen und b) Verwendung des bei a) erhaltenen Absorptionswertes oder der -werte zur Erzeugung eines Outputsignals, welches für den Gehalt an aromatischen Verbindungen des Gemisches indikativ ist, **gekennzeichnet** durch die Stufen:

    i) Bereitstellung einer Vorprobe mit der gleichen Zusammensetzung wie das zu analysierende Gemisch oder mit einer dazu ähnlichen Zusammensetzung;
    ii) Trennung der Vorprobe in eine aromatische Fraktion und eine nichtaromatische Fraktion;
    iii) Messung der Infrarotabsorption dieser Fraktionen jeweils bei der gleichen ausgewählten Wellenlänge oder den gleichen ausgewählten Wellenlängen im Bereich von 1650 bis 1700 nm oder 2120 bis 2256 nm;
    iv) Verwendung der bei iii) gemessenen Absorptionswerte zur Ableitung einer Eichgleichung, die bei der darauffolgenden Analyse verwendet wird;
    v) Messung der Infrarotabsorption des zu analysierenden Gemisches bei der gleichen ausgewählten Wellenlänge oder den gleichen ausgewählten Wellenlängen; und
    vi) Verwendung der Eichgleichung, die bei der Stufe iv) abgeleitet wurde, zur Bestimmung des aromatischen Gehaltes des Gemisches.

2. Verfahren nach Anspruch 1, wobei bei der Stufe i) des Vor-Eichverfahrens die Probe die gleiche Zusammensetzung wie das zu analysierende Gemisch besitzt und durch Entnahme einer Vorprobe aus dem Gemisch erhalten worden ist.

3. Verfahren nach Anspruch 1, wobei das Vor-Eichverfahren unter Verwendung einer Probe durchgeführt wird, die aus einem unterschiedlichen Kohlenwasserstoff-Gemisch als das zu analysierende Gemisch entnommen wurde, aber trotzdem eine ähnliche Zusammensetzung aufweist.

4. Verfahren nach einem der Ansprüche 1 bis 3, wobei bei der Stufe iv) die bei der Stufe iii) erhaltenen Absorptionswerte zur Ableitung der Eichgleichung durch Anwendung des Beer-Lambert-Gesetzes verwendet werden.

5. Verfahren nach einem der Ansprüche 1 bis 4, wobei bei der Stufe a) des Meßverfahrens und der Stufe iii) des Vor-Eichverfahrens die Absorptionen bei einer einzigen Wellenlänge innerhalb der Bereiche 1650 bis 1700 nm und 2120 bis 2256 nm gemessen werden.

6. Verfahren nach Anspruch 5, worin die einzige Wellenlänge 1672 nm beträgt.

7. Verfahren nach einem der Ansprüche 1 bis 6, wobei bei der Stufe ii) des Eichverfahrens die Probe in aromatische und nichtaromatische Fraktionen durch Hochleistungs-Flüssigkeitschromatographie getrennt wird.

8. Verfahren nach einem der Ansprüche 1 bis 7, wobei die Absorptionsmessung der Stufe a) auf einer im wesentlichen kontinuierlichen Basis durchgeführt wird, während das zu analysierende Kohlenwasserstoff-Gemisch oder ein Teil davon durch eine Strömungszelle fließt, die so gebaut ist, daß sie die Messung der Infrarotabsorptionen mit einer Flüssigkeit, die durch diese Zelle fließt, erlaubt, wobei das bei der Stufe b) erzeugte Signal somit im wesentlichen der momentane Gehalt an aromatischen Verbindungen des flüssigen Kohlenwasserstoff-Gemisches, so wie es durch die Zelle fließt, ist.

9. Verfahren zur Behandlung eines flüssigen Kohlenwasserstoff-Beschickungsmaterials, das die Behandlung des Beschickungsmaterials in einer Weise umfaßt, die den Gehalt an aromatischen Verbindungen in dem Beschickungsmaterial beeinflußt, wobei der Gehalt an aromatischen Verbindungen des Beschickungsmaterials vor und nach der Behandlung gemäß dem in Anspruch 8 beschriebenen Verfahren verfolgt bzw. analysiert wird und wobei einer oder mehrere Verfahrensparameter, der bzw. die den Gehalt an aromatischen Verbindungen in dem Beschickungsmaterial nach der Behandlung beeinflußt bzw. beeinflussen, in Antwort auf das Outputsignal eingestellt wird bzw. werden, welches für den momentanen aromatischen Gehalt des Beschickungsmaterials zum Zeitpunkt der Messung indikativ ist.

10. Verfahren nach Anspruch 9, welches die Hydrobehandlung eines flüssigen Kohlenwasserstoff-Beschickungsmaterials umfaßt, um den Gehalt an aromatischen Verbindungen zu verringern, und wobei die Verfolgung bzw. Analyse mit dem aus der Hydrobehandlungsvorrichtung abfließenden Material erfolgt, wobei das Outputsignal, das für den aromatischen Gehalt des abfließenden Materials indikativ ist, verwendet wird, um einen oder mehrere der Parameter, der bzw. die die Hydrobehandlung beeinflußt bzw. beeinflussen, einzustellen und somit das Hydrobehandlungsverfahren zu kontrollieren.

11. Verfahren nach Anspruch 10, angewendet für die Kontrolle der Cetanzahl eines Dieselkraftstoffs, der durch Hydrobehandlung von Dieselkraftstoff-Beschickungsmaterial gebildet wird.

**Revendications**

1. Un procédé pour la mesure de la teneur en aromatiques d'un mélange d'hydrocarbures liquides contenant à la fois des composés aromatiques et des composés non aromatiques, qui comprend les opérations consistant à a) mesurer l'absorbance à l'infrarouge proche du mélange à une ou à des longueurs d'onde choisies et b) utiliser la ou les valeurs d'absorbance obtenues en a) pour produire un signal de sortie indicateur de la teneur en aromatiques du mélange, caractérisé par les opérations consistant à

   i) obtenir un échantillon préliminaire présentant la même composition que le mélange à analyser, ou une composition analogue à celle-ci ;

   ii) séparer cet échantillon préliminaire en une fraction aromatique et une fraction non aromatique ;

   iii) mesurer l'absorbance a l'infrarouge de ces fractions, chacune à la même longueur d'onde ou aux mêmes longueurs d'onde choisies dans la gamme de 1650 à 1700 nm ou 2120 à 2256 nm ;

   iv) utiliser les valeurs d'absorbance mesurées en iii) pour en déduire l'équation d'étalonnage à utiliser dans l'analyse ultérieure ;

   v) mesurer l'absorbance à l'infrarouge du mélange à analyser à la même ou aux mêmes longueurs d'onde choisies ; et

   vi) utiliser l'équation d'étalonnage obtenue à l'opération iv) pour déterminer la teneur en aromatiques de ce mélange.

2. Un procédé selon la revendication 1, dans lequel, dans l'opération i) du processus d'étalonnage préalable, ledit échantillon est de la même composition que le mélange à analyser et est obtenu par un échantillonnage préalable de ce mélange.

**3.** Un procédé selon la revendication 1, dans lequel le processus d'étalonnage préalable est effectué en utilisant un échantillon pris à partir d'un mélange d'hydrocarbures différent du mélange qui est à analyser mais présentant néanmoins une composition analogue.

**4.** Un procédé selon une quelconque des revendications 1 à 3, dans lequel, dans l'opération iv), les valeurs d'absorbance obtenues dans l'opération iii) sont utilisées pour en déduire l'équation d'étalonnage par l'application de la loi de Beer-Lambert.

**5.** Un procédé selon une quelconque des revendications 1 à 4, dans lequel, dans l'opération a) du processus de mesure et dans l'opération iii) du processus d'étalonnage préalable, les absorbances sont mesurées à une longueur d'onde unique à l'intérieur desdites gammes de 1650 à 1700 nm et 2120 à 2256 nm.

**6.** Un procédé selon la revendication 5, dans lequel cette longueur d'onde unique est 1672 nm.

**7.** Un procédé selon une quelconque des revendications 1 à 6, dans lequel, dans l'opération ii) du processus d'étalonnage, ledit échantillon est séparé en une fraction aromatique et en une fraction non aromatique par une chromatographie en phase liquide à haut rendement.

**8.** Un procédé selon une quelconque des revendications 1 à 7, dans lequel la mesure d'absorbance de l'opération a) est effectuée sur une base sensiblement continue alors que le mélange d'hydrocarbures à analyser, ou une partie de ce dernier, s'écoule à travers une cellule d'écoulement conçue pour permettre la mesure des absorbances à l'infrarouge sur un liquide s'écoulant à travers cette cellule, le signal produit dans l'opération b) représentant ainsi une teneur en aromatiques sensiblement instantanée du mélange d'hydrocarbures liquides au fur et à mesure qu'il s'écoule à travers cette cellule.

**9.** Un procédé de traitement d'une charge d'hydrocarbures liquides qui implique le traitement de la charge de manière à affecter la teneur en aromatiques de cette charge, dans lequel la teneur en aromatiques de la charge, avant ou après ledit traitement, est contrôlée au moyen d'un processus revendiqué à la revendication 8, et dans lequel un ou plusieurs paramètres du processus affectant la teneur en aromatiques de la charge, après traitement, sont réglés en réponse au signal de sortie indicatif de la teneur instantanée en aromatiques de la charge au point de mesure.

**10.** Un procédé selon la revendication 9, qui implique l'hydrotraitement d'une charge d'hydrocarbures liquides de manière à réduire sa teneur en aromatique, et dans lequel ledit contrôle est effectué sur l'effluent sortant du dispositif d'hydrotraitement, le signal de sortie indicatif de la teneur en aromatiques de cet effluent étant utilisé pour régler un ou plusieurs des paramètres affectant l'hydrotraitement et pour commander ainsi le processus d'hydrotraitement.

**11.** Un procédé selon la revendication 10, tel qu'appliqué à la commande de l'indice de cétane d'un combustible diesel, qui est produit par l'hydrotraitement d'une charge de combustibles diesel.

FIG. 1

FIG. 2

FIG. 3

300 310 320 330 340 350 360 370 380

FIG. 4

EP 0 561 789 B1

FIG. 5

EP 0 561 789 B1

FIG. 6

FIG. 7

14

FIG. 8